# FASCICULE DE BREVET EUROPEEN

(11) **EP 2 705 369 B1**
(45) Date de publication et mention de la délivrance du brevet: **12.09.2018**
(21) Numéro de dépôt: 12725057.9
(22) Date de dépôt: 26.04.2012
(51) Int. Cl.: G01N 33/569, G01N 33/68

(54) **PROCÉDÉ POUR LA DÉTECTION D'UNE INFECTION PAR LE VIRUS DE LA DENGUE**
VERFAHREN FÜR DEN NACHWEIS EINER INFEKTION DURCH DAS DENGUE-VIRUS
METHOD FOR THE DETECTION OF AN INFECTION BY THE DENGUE VIRUS

(30) Priorité: 06.05.2011 FR 1153916
(43) Date de publication de la demande: 12.03.2014
(73) Titulaire: Biomerieux, 69280 Marcy-l'Étoile (FR)
(72) Inventeur: BEDIN, Frédéric, F-69007 Lyon (FR); BUSSERET, Sandrine, F-69001 Lyon (FR); STEIDEL, Marine, F-68470 Fellering (FR)
(86) Numéro de dépôt international: PCT/FR2012/050926
(87) Numéro de publication internationale: WO 2012/153031

(56) Documents cités:
- WO-A2-2010/043973
- FR-A1- 2 794 864

## Description

La présente invention a pour objet un procédé pour la détection et le sérotypage précoce du virus de la dengue.

Ces 30 dernières années, la dengue, une maladie virale transmise par les moustiques hématophages urbains du genre *Aedes,* s'est répandue à travers le monde de manière inquiétante. C'est actuellement un véritable problème de santé publique pour plus d'une centaine de pays situés dans la zone subtropicale, particulièrement dans les zones pacifique-ouest, Amérique du sud et Asie du sud-est. L'émergence de la maladie est due en grande partie à l'explosion démographique et à l'urbanisation anarchique. Les anomalies climatiques ont également un rôle non négligeable. A ce titre, la dengue pourrait émerger dans les régions occidentales du globe jusqu'alors épargnées par le virus. Ainsi, *Aedes albopictus,* un des vecteurs de la maladie, a récemment été trouvé dans le nord de l'Italie et dans le sud de la France. Dernièrement, des cas de dengue autochtones ont été signalés dans le sud de la France. On estime que près de 3 milliards de personnes sont exposés aux risques de dengue. Près d'un million d'hospitalisations sont recensées annuellement et les décès se comptent par milliers. Les enfants sont les principales victimes de la maladie.

Le virus de la dengue est un virus enveloppé à ARN monocaténaire de polarité positive de la famille des Flaviviridae. Le génome du virus (11 000 nucléotides) code pour une polyprotéine d'environ 3400 acides aminés qui subit un clivage co- et post-traductionnel qui résulte en des protéines structurales (C, prM, E) et des protéines non-structurales (NS1, NS2A, NS2B, NS3, NS4A, NS4B, NS5). Il existe 4 sérotypes viraux (DV1 à DV4), qui peuvent coexister en zones d'endémie. Il y a environ 70% d'homologie de séquences entre les différents sérotypes. L'infection par un sérotype donné confère une immunité sur le long terme pour ce sérotype. La protection croisée ne dure que quelques mois : la réinfection est donc possible avec un sérotype différent. La manifestation clinique la plus commune (fièvre de dengue ou DF) est un état fébrile de quelques jours accompagné notamment de céphalées sévères, de lombalgies, de douleurs musculaires et articulaires, qui régressent spontanément sans traitement spécifique. Cependant, on assiste parfois a des complications conduisant à une dengue hémorragique (ou DHF). Dans ce cas, on note une augmentation transitoire de la perméabilité vasculaire et une fuite du plasma responsable d'une thrombopénie et d'une coagulopathie. Dans les cas les plus sévères, la fuite de plasma peut entraîner un choc hypovolumique mortel (DSS, Dengue Shock Syndrome) si le patient n'est pas pris en charge rapidement. Des atteintes hépatiques et neurologiques, rares mais mortelles, sont également associées à la sévérité de la maladie. Variable selon les épidémies, le taux de mortalité peut atteindre 5% des cas déclarés de DHF. Ce taux peut augmenter jusqu'à 20% sans une prise en charge hospitalière ou traitements adaptés.

90% des cas de DHF ont lieu lors d'une infection secondaire par un sérotype hétérologue et 10% lors d'une infection primaire, habituellement chez des nourrissons âgés de 6 mois à 1 an. Il existe plusieurs facteurs qui influent sur la sévérité de l'infection, tels que les facteurs de l'hôte, le sérotype et le génotype du virus, l'ordre de succession des virus infectants, la qualité et la quantité d'anticorps à réactions croisées et la réponse CD4/CD8. Les causes exactes de l'apparition de la DHF ne sont cependant toujours pas connues avec certitude. Dans une première hypothèse, ce sont les forces de sélection exercées sur le virus qui conduisent à la sélection d'un « super-virus ». Des études ont montré une corrélation entre la charge virale et la sévérité de la maladie. Des études ont également impliqué les protéines virales E et NS1 dans la pathogénicité. La séquence des sérotypes infectants, l'intervalle entre chaque infection sont également des déterminants cliniques importants. Ainsi, une infection secondaire par les sérotypes 1 et 2 est souvent associée à une fréquence élevée de DHF. Jusqu'à présent, aucun déterminant spécifique de la virulence n'a été mis en évidence avec certitude. Une infection secondaire avec un sérotype différent est significativement associée à la sévérité de la maladie. C'est la base de la deuxième hypothèse, l'hypothèse de la facilitation de l'infection par des anticorps anti-dengue de faible affinité, qui n'a jamais pu être confirmée *in vivo* en l'absence de modèle animal. Elle repose sur la présence d'anticorps neutralisants de faible affinité qui facilitent l'infection *in vitro* des macrophages via leur récepteur Fc aux immunoglobulines. La présence des complexes immuns favorise par ailleurs l'activation du complément. L'activation et la prolifération excessive des lymphocytes T CD4 et CD8+ mémoires entraîneraient la production accrue de cytokines et de médiateurs cellulaires. La concomitance de ces différents éléments serait responsable de la pathogénie. Si la mise en évidence du rôle clef de l'activation lymphocytaire et de la sécrétion de médiateur dans la physiopathologie est assez bien admise, il est très difficile de montrer une corrélation claire avec l'apparition de la DHF.

Les facteurs génétiques jouent probablement un rôle puisque plusieurs études ont montrées le rôle soit protecteur soit pathogène de certains allèles HLA classe 1. Cependant, les résultats sont assez contradictoires et varient suivant l'origine des prélèvements.

A l'heure actuelle, il n'existe pas de vaccins commercialisés contre le virus de la dengue et les seuls traitements disponibles sont des traitements symptomatiques. Dans ce contexte, il est important de pouvoir surveiller les épidémies et de pronostiquer les cas sévères pour une prise en charge hospitalière adaptée^{[1, 2, 3]}.

Le diagnostic de la dengue est basé sur la détection des éléments viraux ou des anticorps spécifiques. Parmi les techniques de virologie classiques, l'isolement du virus par infection de cellules et examen par immunofluorescence reste une des techniques de référence. Cependant, elle est techniquement très lourde à mettre en oeuvre.

Les méthodes moléculaires (RT-PCR...) permettent la détection du virus et le sérotypage. Parmi les méthodes sérologiques, le MAC-ELISA (*Immunoglobulin M Antibody Capture ELISA)* qui mesure les IgM spécifiques, est utilisé pour détecter les infections primaires. Toutefois, elles nécessitent d'être associées à une détection des IgG spécifiques pour discriminer infections primaires et secondaires. Mais, les anticorps n'apparaissent que 4-5 jours après le début des symptômes et de plus il est nécessaire de disposer de deux prélèvements de façon à mettre en évidence une séroconversion.

La protéine NS1 a été caractérisée en 1985 par G.W Smith^{[4]}. C'est une glycoprotéine hautement conservée chez les flavivirus. Bien que sa fonction ne soit pas entièrement élucidée, elle interviendrait notamment dans la réplication du virus et dans la pathogénicité. Elle est synthétisée sous forme de monomère. Sa maturation est associée à une dimérisation. La protéine mature est transportée vers la membrane plasmique puis excrétée dans le milieu extracellulaire où elle est retrouvée sous forme de dimères et de multimères, principalement des pentamères et des hexamères^{[6]}.

La recherche de protéine NS1 dans des prélèvements sanguins de patients permet de déterminer une infection récente mais ne permet pas de distinguer entre dengue primaire et secondaire, élément important, puisque la DHF est majoritairement associée à une seconde infection par un sérotype différent.

Des méthodes de type ELISA ont été développées pour mettre en évidence la présence de la protéine virale NS1 et diagnostiquer une dengue dès l'apparition de la fièvre (voir p.ex. FR2794864 A1). Dans ces méthodes, l'utilisation de mélanges d'anticorps monoclonaux et/ou d'anticorps polyclonaux sélectionnés pour leur affinité vis-à-vis de la protéine NS1 permettent une détection précoce, pendant la phase clinique de l'infection.

Le problème de ces méthodes ELISA est qu'elles nécessitent d'utiliser au moins deux anticorps, un anticorps de capture et un anticorps de détection dirigés contre des épitopes différents de la protéine, ce qui implique des contraintes dans la sélection des matières premières et des contraintes techniques dans la réalisation de l'essai. De plus, ces méthodes ne permettent pas de distinguer les différents sérotypes, alors qu'il est établi qu'une infection secondaire par les sérotypes 1 et 2, par exemple, est souvent associée à une fréquence élevée de DHF.

Aussi, il est important de disposer d'une méthode de détection plus simple qui permette de détecter précocement la protéine NS1 dans un échantillon pour établir le diagnostic d'une infection qu'elle soit primaire ou secondaire et de plus de distinguer les différents sérotypes, en particulier les sérotypes associés à une fréquence élevée de DHF.

WO2010/043973 concerne des biomarqueurs à base de protéine et des combinaisons de biomarqueurs que l'on utilise pour qualifier l'état de la dengue chez un patient. Plus précisément, les biomarqueurs sont utilisés pour classer un échantillon objet comme étant infecté ou non par la dengue. Les biomarqueurs peuvent être détectés par spectrométrie de masse SELDI.

La présente invention répond aux problématiques discutées ci-dessus par un procédé qui permet à la fois une détection précoce et spécifique de la protéine NS1 dans un échantillon biologique et de distinguer les différents sérotypes.

Aussi, la présente invention a notamment pour objet un procédé pour mettre en évidence *in vitro* une infection par un virus de la dengue chez un individu qui comprend les étapes de :
mettre en contact un échantillon sanguin de l'individu avec un ligand spécifique de la protéine NS1 dudit virus de la dengue pour capturer la protéine NS1, si elle est présente dans l'échantillon sanguin, ledit ligand étant immobilisé sur un support solide,
mettre en évidence la présence de la protéine NS1 par une lecture par spectrométrie de masse, et
si la protéine NS1 est mise en évidence conclure que l'individu a été infecté par le virus de la dengue.

La spectrométrie de masse est une méthode qui utilise un spectromètre de masse pour détecter des ions en phase gazeuse. Un spectromètre de masse est constitué d'une source pour ioniser à l'état gazeux les molécules à analyser, par exemple un laser, et d'un analyseur pour déterminer la nature de la masse en utilisant différents principes physiques. A titre d'exemple, on peut citer comme principes physiques le temps de vol ou TOF (Time Of Flight), le piège à ions, la résonance ionique cyclotronique, le filtre quadripole, le secteur magnétique, le secteur électrostatique.

Les spectromètres de masse de type MALDI-TOF sont des spectromètres de masse couplant une source d'ionisation laser assistée par une matrice (MALDI : *Matrix-Assisted laser Desorption*/*Ionisation*) à un analyseur à temps de vol (TOF). Historiquement, les analyseurs par temps de vol, qui nécessitent une source d'ionisation pulsée, étaient couplés uniquement avec les sources MALDI. Actuellement, les sources MALDI peuvent être couplées à d'autres types d'analyseurs (par exemple un analyseur FT-ICR) et l'analyseur par temps de vol à d'autres sources (par exemple une source électrospray dans un instrument ESI-QTOF).

Le SELDI-TOF MS (*Surface Enhanced Laser Desorption*/*Ionisation Time Of Flight Mass Spectrometry)* est une approche mise au point par Hutchens et al.^{[7]}. Dans cette technique, des protéines sont retenues sur des surfaces chromatographiques disposées sur des barrettes et sont ensuite ionisées par la technique MALDI et détectées par TOF MS (Time Of Fly Mass Spectrometry). Les surface chromatographiques sont conçues pour sélectionner les protéines présentes dans un mélange complexe, sur des critères d'hydrophobicité, de charge, d'affinité etc.... La masse moléculaire des protéines retenues par les surfaces chromatographiques peut être mesurée par TOF MS.

Ainsi, dans un mode de réalisation particulier de l'invention on met en contact l'échantillon sanguin de l'individu avec un ligand spécifique de la protéine NS1 dudit virus de la dengue pour capturer la protéine NS1, si elle est présente dans l'échantillon, le ligand étant immobilisé sur un support solide,
on soumet le support à une lecture par spectrométrie de masse pour mettre en évidence la présence de la protéine NS1, et
si la protéine NS1 est mise en évidence on conclut que l'individu a été infecté par le virus de la dengue.

Outre le fait que la méthode de détection par spectrométrie de masse, en particulier MALDI-TOF et SELDI-TOF, permet de différencier les différents sérotypes , elle présente de plus l'avantage de ne nécessiter l'utilisation que d'un seul ligand spécifique en capture, pour une spécificité au moins comparable à celle obtenue par une méthode d'ELISA classique utilisant au moins deux anticorps spécifiques dirigés contre deux épitopes différents (un anticorps de capture et un anticorps de détection) et pour une sensibilité voisine de celle obtenues par une méthode ELISA.

Le ligand spécifique de la protéine NS1 est choisi parmi les anticorps, les fragments d'anticorps et les protéines d'affinité aux propriétés compétitives (les nanofitines™). De préférence le ligand est un anticorps tel que défini dans le paragraphe « Définitions », en particulier un anticorps monoclonal ou un anticorps polyclonal hautement purifié par affinité vis-à-vis de la protéine NS1. Par exemple, le ligand est un anticorps monoclonal spécifique de la forme monomérique, de la forme dimérique et de la forme hexamérique de la protéine NS1.

Le ligand est spécifique d'au moins un sérotype du virus de la dengue, en particulier de deux sérotypes du virus.

En particulier le ligand est immobilisé sur un support tel que : une barrette, une plaque, une bille, une puce, une phase chromatographique.

### Définitions

Echantillon sanguin signifie sang total, sérum ou plasma.

Le ligand est de préférence un anticorps, par exemple un anticorps monoclonal ou un anticorps polyclonal hautement purifié polyclonal vis-à-vis de la protéine NS1 ou une protéine d'affinité aux propriétés compétitives (nanofitine™)
Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Les nanofitines (nom commercial) sont de petites protéines qui comme les anticorps sont capables de se lier à une cible biologique permettant ainsi de la détecter, de la capturer ou tout simplement de la cibler au sein d'un organisme.

### Figures

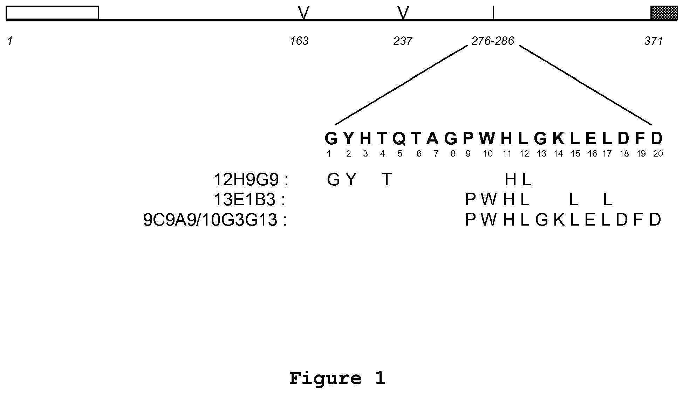

La figure 1 représente les séquences d'acides aminés reconnues par les anticorps 12H9G9, 13E1B3, 10G3G13 et 9C9A9. Le gène NS1 synthétique est illustré avec son peptide signal (peptide signal de l'activateur du plasminogène tissulaire - rectangle blanc) et sa queue polyhistidine (rectangle noir) nécessaires, respectivement, à l'excrétion et à la purification de NS1. Les acides aminés identifiés par cartographie d'épitopes sont identifiés sous la séquence spécifique numérotée de 1 à 20 (anticorps 12H9G9, 13E1B3, 10G3G13 et 9C9A9). Les sites potentiels de *N*-glycosylation sont identifiés par « V ».

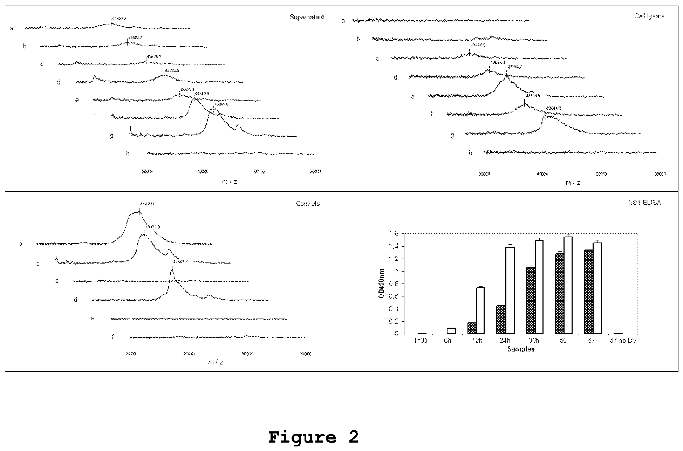

La figure 2 illustre la cinétique de détection de NS1 en SELDI-TOF MS avec l'anticorps 13E1B3 sur des cellules Vero en culture infectées par le sérotype 3 du virus de la dengue. Les résultats sont présentés séparément pour le surnageant de culture et pour le lysat cellulaire. La figure 2A illustre les résultats obtenus sur surnageant de culture, la figure 2B représente les résultats obtenus sur lysat cellulaire et la figure 2C illustre les résultats obtenus avec les contrôles. Les figures 2A et 2B illustrent les résultats des mesures effectuées respectivement à 90 minutes (a) ; 6 heures (b), 12 heures (c), 24 heures (d), 48 heures (e) ; 5 jours après infection (f), 7 jours après infection (g) ; contrôle sans virus, 7 jours après infection. La valeur associée aux pics correspond à la masse rapporté à la charge (m/z) après calibration avec un standard interne. La figure 2C illustre les résultats des mesures obtenus sur des contrôles complémentaires : (a) protéine recombinante NS1 (50ng), (b) surnageant de culture de cellules Vero infectées par le virus de la dengue de sérotype 3 (7 jours après infection), (c) surnageant de culture de cellules Vero non infectées, (d) lysat de cellules Vero infectées par le virus de la dengue de sérotype 3 (7 jours après infection), (e) lysat de cellules Vero non infectées et (f) milieu de culture. La valeur associée aux pics correspond à la masse rapporté à la charge (m/z) après calibration avec un standard interne. La figure 2D illustre les résultats obtenus par un procédé de type sandwich ELISA. Les histogrammes illustrent les ELISAs de capture de la NS1 effectués sur les lysats de cellules Vero (en blanc) et les surnageant de culture (en noir) de ces mêmes cellules prélevées respectivement à 90 minutes, 6, 12, 24, 48 heures, 5 et 7 jours après infection. Les valeurs correspondent à la densité optique (OD) lue à 450 nm (moyenne de 2 puits pour 2 expériences indépendantes).

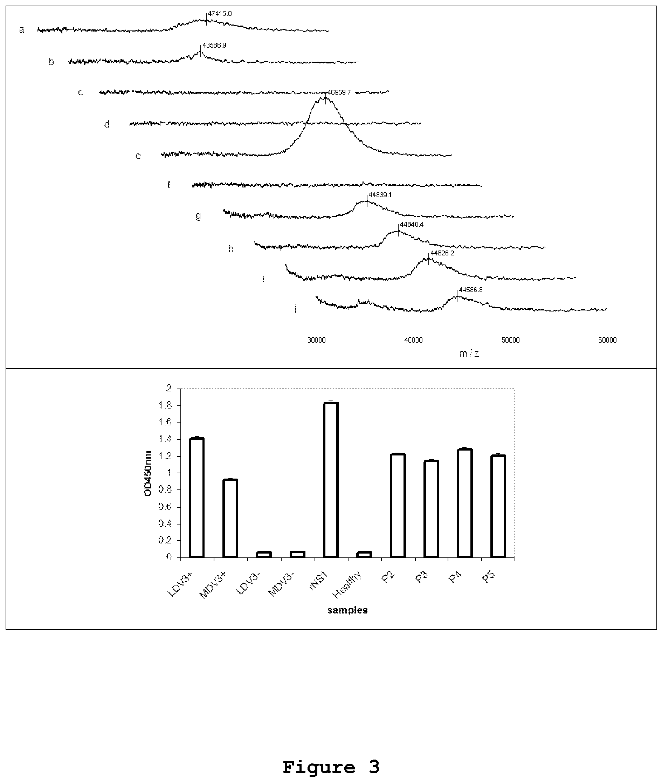

La figure 3 illustre la détection de la protéine NS1 en SELDI-TOF MS sur des plasmas (sérotypes 3) prélevés à différents temps après le début des symptômes.; a) surnageant de culture de cellules Vero infectées par le virus de la dengue de sérotype 3 (2 jours post-infection) ; b) lysat de cellules Vero infectées par le virus de la dengue de sérotype 3 (2 jours post-infection) ; c) surnageant de culture de cellules Vero non infectées ; d) lysat de cellules Vero non infectées ; e) protéine recombinante NS1 (50ng) ; f) plasma sain (« healthy ») ; (g à j) prélèvements sériés de plasmas atteints de dengue à 2, 3, 4, 5 jours après l'apparition des symptômes (p2 à p5). La valeur associée aux pics correspond à la masse rapporté à la charge (m/z) après calibration avec un standard interne. Les histogrammes illustrent des ELISAs de capture de la NS1 effectués sur les mêmes échantillons. Les valeurs correspondent à la densité optique lue à 450nm (moyenne de 2 puits pour 2 expériences indépendantes).

### Exemples

### Exemple 1 : expression et purification d'une protéine NS1 recombinante

Le gène NS1 du virus de la dengue a été obtenu en reconstituant par oligonucléotides chevauchants un gène synthétique de la protéine NS1 en tenant compte des acides aminés présents sur les sérotypes 3 et 1 (http://www.ncbi.nlm.nih.gov/genomes/VirusVariation/). Les codons ont été optimisés pour une expression dans des système d'expression eucaryotes. Un peptide leader correspondant au peptide leader de l'activateur du plasminogène tissulaire a été rajouté en 5' du gène afin de permettre une excrétion de la protéine synthétisée^{[8]}. Une queue poly-histidine a été rajoutée en 3', afin de faciliter la purification de la protéine synthétisée par une méthode d'affinité métal-chélate. Des sites de restriction *Xho*1 et *Xba*1 ont également été ajoutés respectivement en 5' et 3' du gène. Le gène ainsi reconstitué a été inséré aux sites *Xho*1*-Xba*1 d'un plasmide d'expression eucaryotique de type pCI (Promega-Biotech) sous la dépendance d'un promoteur de type « gènes précoces » du CytoMegaloVirus. La protéine NS1 est exprimée grâce à une technique d'expression transitoire dans le système cellulaire eucaryote 293T (ATCCIDCRL-11268) : des cellules 293T à 70% de confluence sont mises en contact avec le plasmide recombinant pCI-NS1 en présence d'un transfectant (Lipofectamine 2000, Invitrogen) selon les instructions du fabriquant. Soixante-douze heures après transfection, le surnageant de culture est récupéré et centrifugé à basse vitesse pour éliminer les débris cellulaires (1500g/5minutes). Le surnageant préclarifié est alors purifié. Il est mis en contact la nuit à 6°C, sous agitation, avec une résine NiNTA (Qiagen). Le lendemain, la résine est lavée puis la protéine NS1 est éluée en présence de tampon salin contenant 250µM d'imidazole selon les recommandations de *The Quiaexpressionist* (*A hanbook for high level expression and purification of his-tagged protein,* fifth édition Qiagen). L'imidazole est ensuite éliminé par une dialyse sur la nuit contre du Tris-Buffer Saline pH=7.4 (TBS) dans une cassette de dialyse de porosité 3500 MWCO (Slide-A-Lyzer, Thermo-Scientific). La protéine dialysée est éventuellement concentré par passage sur une nacelle de type Ultrafree (Millipore). La protéines obtenue est d'une pureté supérieure à 90%. La taille estimée en western-blot, révélé avec un anticorps anti-polyhistidine (PentaHis antibody, Qiagen) après migration sur un gel de polyacrylamide 10% en conditions dénaturantes, est d'environ 45 000 Daltons, soit la taille attendue pour la protéine monomérique. Une hydrolyse des *N*-glycanes par la PNGaseF (New England Biolabs) suivie d'une électrophorèse sur gel de polyacrylamide 10% en conditions dénaturantes confirme, par une baisse de la masse moléculaire apparente, que la protéine obtenue est bien glycosylée. La protéine est quantifiée par micro-BCA (Thermo-Scientific) .

### Exemple 2 : immunisations des souris et des lapins et obtention des anticorps

**Immunisation des souris :** le protocole d'immunisation des souris a consisté en 5 injections de 5 souris Balb/C femelles avec 50 microgrammes de protéine NS1 recombinante purifiée selon l'exemple 1 en présence d'adjuvant complet (première injection) puis incomplet de Freund, par voie sous-cutanée. L'intervalle entre chaque injection est de 3 à 4 semaines. A l'issue du protocole d'immunisation, les cellules de rate des souris sont fusionnées avec un myélome murin et mise en culture jusqu'à apparition des clones selon le protocole standard^{[9]}.
Les hybridomes sécrétant les anticorps anti-NS1 sont sélectionnés par ELISA : la protéine recombinante NS1, obtenue selon l'exemple 1, est fixée par adsorption sur une plaque 96 puits à la concentration de 1µg/mL en tampon Tris-maléate pH=6.2 pendant une nuit à 6°C. Après 3 lavages au TBS-Tween 0.1% la protéine est incubée avec les surnageants des différents hybridomes dilués au ½ en TBS-Tween 0,1%-Gélatine 0.1% (TBS-TG) 1 heure à 37°C. Après 3 nouveaux lavages, un conjugué anti-IgG de souris marqué à la peroxydase de raifort dilué en TBS-TG est ajouté et incubé 45 minutes à 37°C avant les derniers lavages puis la révélation par le kit 1-step Turbo-TMB (Thermo-Scientific). Un surnageant de cellules 293T non transfectées sert de témoin de signal non spécifique. Les immunoglobulines des clones positifs sont purifiés par immuno-affinité sur protéine A sépharose 4FF (GE Healthcare) selon le protocole classique. Sept anticorps monoclonaux ont finalement été sélectionnés sur la base de ce protocole : 10E2H2, 10G3G13, 13E1B3, 12D2D6, 9C9A9, 12H9G9 et 6H10B9.

**Immunisation des lapins** : Le protocole a consisté à injecter à 3 lapins New Zealand White femelles, 5 doses de 200 µg de protéine NS1 recombinante chacune. La protéine NS1 à été obtenue comme décrit dans l'exemple 1. Les trois premières injections étaient intradermiques, les deux suivantes sous-cutanées. L'adjuvant complet (première injection) puis incomplet de Freund a été ajouté à la protéine. Un délai de 2 semaines a été respecté entre chaque injection. Les lapins sont finalement saignés 82 jours après la première injection et 60mL de sérum sont récupérés. Le titre La présence des anticorps spécifiques est vérifié selon l'ELISA décrit dans l'exemple 2 (immunisation de souris). Les sérums des lapins prélevés avant immunisation servent de témoin de spécificité. Les immunoglobulines G sont purifiées sur colonne de protéine A Sépharose. Le titre de ces anticorps purifiés a été estimé en ELISA par une dilution sériée de l'anticorps sur de la protéine recombinante NS1 coatée en fond de puits d'une plaque 96 puits et après révélation par un anticorps anti-lapin couplé à la peroxydase et dilué au 1/5000 à l'aide du kit 1-step Turbo-TMB (Thermo-Scientific). Le titre, qui correspond à la plus forte dilution pour laquelle la densité optique est trois fois supérieure au bruit de fond obtenu avec les sérums des lapins pré-immuns, est estimé à 10⁵.

### Exemple 3 : caractérisation des anticorps monoclonaux et polyclonaux

### Matériel et Méthode

Les anticorps ont été caractérisé par différentes approches : (i) western-blot et ELISA sur la protéine recombinante NS1 ou sur du matériel infecté par le virus de la dengue ; (ii) cartographie d'épitopes (Spotscan et Phage Display).
(i) Pour le Western-blot on utilise une protéine NS1 recombinante dénaturée et réduite par la présence de SDS et de β-Mercapto-Ethanol dans le tampon de charge, à une concentration finale en protéine totale de 0,1 mg/ml. Le volume de dépôt est de 20 µl par puits, sur un gel NuPAGE Novex Bis-Tris 4-12%, tampon de migration MOPS (InVitrogen). Après migration (sous 200V, pendant 1 heure) et transfert sur membrane de PVDF (sous 400 mA, pendant 45 min), la qualité du transfert est appréciée par une coloration à l'amidoblack. Les membranes sont saturées par 5% de lait écrémé (Régilait) dans une solution de TNT (Tris 15 mM, NaCl 0,14M, Tween 20 0,5% pH8) à température ambiante pendant 1 heure. Après saturation, les membranes sont incubées pendant 1 heure avec les différents anticorps à tester dilués à 5 µg/ml dans la solution de saturation. Après rinçages au TNT, les membranes sont incubées 1 heure à température ambiante avec un conjugué anti-souris-phosphatase alcaline dilué au 1 : 5000 (Jackson Immunoresearch) dans la solution de saturation. Après rinçage, la révélation est réalisée avec le kit 1-STEP NBT/BCIP (Thermo-scientific) suivant les données d'utilisation recommandées.
   NS1 a pu également être détecté en immunoessai de type sandwich. Pour ce faire, les plaques 96 puits (Nunc) ont été coatées avec les anticorps monoclonaux anti-NS1 à tester en capture à 2 µg par puits. Après 3 lavages en TBS-Tween 20 0,5% (TBS-T), les plaques sont saturées par 10% de lait écrémé (Régilait) dilués dans du TBS-T, pendant 1h à 37°C. On lave encore 3 fois en TBS-T, on dépose sur les plaques 100µL d'échantillon à tester, éventuellement dilué en TBS-T 1% BSA et on incube 2h à 37°C. Après 3 lavages TBS-T, l'anticorps polyclonal de lapin anti-NS1 (décrit dans l'exemple 2) dilué au 1/2000ème est rajouté et on incube 2h à 37°C. On effectue encore 3 lavages en TBS-T, avant de rajouter le conjugué couplée à la peroxydase de raifort (Jackson Immunoresearch) dilué au 1/5000 en TBS-T 3% BSA, 100 µl/puits. Après 1h d'incubation à 37°C et 3 lavages en TBS-T, on ajoute le substrat 1-step Turbo-TMB (Thermo-Scientific), 100 µl/puits. Au bout de 20 min, lorsque le développement de la coloration a lieu, on stoppe la réaction par de l'acide sulfurique 1N et on mesure l'absorbance à 450 nm. Les résultats ont été obtenus sous forme de valeurs brutes après soustraction du bruit de fond
(ii) La technique du Spotscan, adaptée d'après Frank et Döring ^{[10]}, permet de synthétiser de façon simultanée un grand nombre de peptides fixés sur membrane de cellulose. Ces peptides reproduisent la séquence de l'antigène cible sous forme de peptides de 8 à 12 acides aminés, se chevauchant de 1 à 4 résidus. Ces peptides sont ensuite mis en contact avec l'anticorps à étudier dans un test colorimétrique de type Blot, et l'identification des peptides immunoréactifs permet de déduire la séquence minimale de l'épitope de l'anticorps et de le localiser précisément sur l'antigène.

La synthèse s'effectue sur une membrane de cellulose portant uniformément des bras en polyéthylène glycol (PEG) d'une longueur de 8 à 10 unités, présentant une fonction NH₂ libre en bout de chaîne. Elle se déroule de l'extrémité C-terminale vers l'extrémité N-terminale des peptides. Les acides aminés ont leur fonction aminée protégée par un groupement Fmoc (9-fluoréméthyloxycarbonyl), et leurs chaînes latérales, susceptibles de réagir au cours de la synthèse, sont également protégées par des groupements trityl, t-butyl ou t-butyl-éther. Les solutions-mères d'acides aminés sont préparées à une concentration de 0,33 M dans du NMP (N-méthyl-pyrrolidone) contenant 0,5 M de HOBt (hydroxybenzotriazole). Le dépôt des acides aminés s'effectue à l'aide du robot ASP 222 (Abimed, Langenfeld, Allemagne), piloté par l'intermédiaire du logiciel AutoSpot XL. L'utilisation de ce robot permet de faire en simultané jusqu'à 4 membranes de 96 spots, soit 384 peptides.

Pour un cycle de couplage d'un acide aminé, le robot dépose 0,7 µl de la solution d'acide aminé activé extemporanément (un volume de solution de diisopropyl-carbodiimide 1,1 M dilué en NMP pour 3 volumes de solution-mère d'acide aminé) sur les membranes. Ce dépôt est répété une seconde fois, puis les membranes sont rincées en DMF (N,N-diméthylformamide). Les groupements NH₂ n'ayant pas réagi sont ensuite été acétylés par 4 à 6 incubations de 10 minutes dans une solution d'anhydride acétique 10 % en DMF, afin d'éviter l'apparition de peptides abortifs ou tronqués. Après 3 lavages de 2 minutes en DMF, les groupements Fmoc protégeant la fonction aminée des acides aminés sont clivés par une incubation de 5 minutes dans une solution de pipéridine 20 % en DMF. Après 4 lavages en DMF, les spots sont colorés à l'aide d'une solution de bleu de bromophénol 1 % en DMF, puis la membrane est rincée 3 fois en méthanol et séchée à l'air libre avant le cycle de couplage suivant.

Ce protocole est répété pour l'ajout de chaque nouvel acide aminé. Après le couplage du dernier acide aminé, les peptides sont acétylés afin de permettre le blocage de tous les groupements NH₂ libres, empêchant ainsi l'ajout d'un autre acide aminé. Puis les chaînes latérales de tous les peptides sont déprotégées par l'incubation des membranes dans un bain acide trifluoroacétique / dichlorométhane / triisobutylsilane
(5 : 5 : 0,3) pendant 1 heure. Les membranes sont ensuite rincées 4 fois en dichlorométhane, 3 fois en DMF et 3 fois en méthanol avant d'être séchées à l'air libre et conservées à -20°C jusqu'à l'immunorévélation.

Pour immunorévéler les spots avec un anticorps monoclonal, les membranes sont d'abord rincées en méthanol, puis lavées en TBS (Tris-HCl 50 mM pH 8,0, NaCl 140 mM, KCl 3 mM) avant d'être incubées sur la nuit à température ambiante dans la solution de saturation (solution concentrée 10X à base de caséine (Western Blocking reagent, Roche) diluée en TBS-Tween 20 0,05% (TBS-T) et contenant 5% de saccharose). Après un lavage de 10 minutes en TBS-T, les membranes sont incubées pendant 1h30 à 37°C avec l'anticorps monoclonal dilué à 20 µg/ml en solution de saturation. Les membranes sont ensuite lavées 3 fois en TBS-T, puis sont incubées avec le conjugué anti-souris couplé à la phosphatase alcaline (Jackson Immunoresearch), dilué au 1/2000^{ème} en solution de saturation. Après 2 lavages de 10 minutes en TBS-T, puis 2 lavages en CBS (acide citrique 10 mM pH 7, NaCl 140 mM, KCl 3 mM), le révélateur, préparé extemporanément (5-bromo, 4-chloro, 3-indoyl, phosphate 600 µM, thiazolyl blue tetrazolium bromide 720 µM, et MgCl₂ 5 mM en CBS), est mis en contact avec la membrane pendant 30 à 45 minutes à l'obscurité. Les peptides immunoréactifs apparaissent en bleu-violet. Après 3 rinçages en eau distillée, les membranes sont scannées puis conservées dans l'eau jusqu'à la régénération.
La régénération permet d'éliminer les anticorps et les conjugués fixés sur les peptides, ce qui permet ainsi de réaliser un nouveau test d'immunoréactivité vis-à-vis d'un autre anticorps. Les membranes subissent une série de lavages de 10 minutes chacun : 1 lavage en eau distillée, 6 lavages en DMF, 3 lavages en tampon de régénération A (urée 8 M, SDS (sodium dodecyl sulfate) 35 mM, β-mercaptoethanol 0,1 %), 3 lavages en tampon de régénération B (eau distillée / éthanol / acide acétique 4 : 5 : 1), puis 2 lavages en méthanol. Les membranes sont ensuite séchées à l'air libre avant d'être stockées à -20°C.

La caractérisation des épitopes par criblage de banques de peptides portés par des phages a été réalisée en utilisant le kit commercial PhD12 Phage Display Peptide Library Kit (Cat. No. E#8110S) de New England Biolabs, en suivant les instructions fournies avec le kit, version 2.7 du protocole datant de novembre 2007.

### Résultats :

**Tableau 1**

| **Anticorps de révélation** | **Formes reconnues de la NS1 recombinante** |
|---|---|
| Anti-tag His (Qiagen) | Monomère + Dimère |
| Mab 10E2H2 | Dimère |
| Mab 10G3G12 | Dimère |
| Mab 13E1B3 | Monomère + Dimère |
| Mab 12D2D6 | Dimère |
| Mab 9C9A9 | Dimère |
| Mab 12H9G9 | Monomère + Dimère |
| Mab 6H10B9 | Monomère + Dimère |
| Polyclonal | Monomère + Dimère |

| | |
|---|---|
| Mab : anticorps monoclonal | |

Le tableau 1 résume les formes monomériques/oligomériques reconnues par les anticorps monoclonaux et l'anticorps polyclonal en Western-blot. Tous les anticorps détectent la forme dimérique mais la forme monomérique est detectée uniquement par les anticorps monoclonaux 13E1B3, 12H9G9, 6H10B9 et le polyclonal de lapin. L'anticorps anti-tag histidine reconnaît les formes monomériques et dimériques. La forme monomérique est présente parce que les échantillons ont été dénaturés au préalable. La forme hexamerique n'est jamais reconnue, car très instable^{[5]}.

**Tableau 2**

| | | **Cellules Vero infectées par DV** | |
|---|---|---|---|
| | **NS1 recomb.** | **lysats** | **surnageants** |
| **10E2H2** | ++ | DV1-DV2-DV3 | X |
| **10G3G13** | + | DV1-DV2-DV3 | DV3 |
| **13E1B3** | +++ | DV1-DV3 | DV1-DV3 |
| **12D2D6** | + | DV1-DV2-DV3 | X |
| **9C9A9** | ++ | DV1-DV2-DV3 | DV3 |
| **12H9G9** | +++ | DV1-DV3 | DV1-DV3 |
| **6H10B9** | + | DV1-DV2-DV3 | X |
| **PA** | +++ | NA | NA |
| **Platelia™** | +++ | DV1-DV2-DV3-DV4 | DV1-DV2-DV3-DV4 |

Le tableau 2 récapitule les différents ELISAs menés sur la NS1 recombinante ou sur le matériel issue de cellules Vero infectées par un des quatre sérotypes du virus de la dengue (DV1, DV2, DV3 ou DV4). L'anticorps polyclonal (PA) obtenu après immunisation de lapins par la protéine NS1, ainsi que le kit Platelia commercialisé par BioRad (Platelia^{tm}) ont également été testés, à titre de comparaison. DVx (où x=1, 2, 3, 4) indique que le signal est considéré comme positif pour le sérotype x avec une densité optique à 450 nm supérieure à 0.1. L'intensité du signal est considérée comme légère (+) quand elle est comprise entre 0.1 et 0.4 de densité optique à 450nm ; elle est considérée comme moyenne (++) entre 0.4 et 0.9 de densité optique et forte (+++) pour une densité optique lue supérieure à 0.9. Les meilleurs signaux obtenus sur la protéine recombinante ont été obtenus avec les anticorps monoclonaux 13E1B3 et 12H9G9 et l'anticorps polyclonal de lapin. Seul les anticorps monoclonaux 10G3G13, 13E1B3, 9C9A9 et 12H9G9 peuvent détecter la NS1 mature, sécrétée dans le surnageant de culture par des cellules Vero infectées par le virus de la dengue. La spécificité est restreinte aux sérotypes 1 et 3 pour les monoclonaux 13E1B3 et 12H9G9. Elle est restreinte au sérotype 3 pour 10G3G13 et 9C9A9.

### Cartographie épitopique :

La cartographie épitopique n'a donné de résultats exploitables que pour les anticorps monoclonaux 13E1B3, 12H9G9, 10G3G12 et 9C9A9. Tous les anticorps sauf 12H9G9 (qui a été cartographié par la technique du *Phage display*) ont été cartographiés par la technique Spotscan. Les résultats obtenus sont illustrés par la figure 1. Pour 13E1B3, 12H9G9, les épitopes sont discontinus. 10G3G12 et 9C9A9 ont le même épitope linéaire. Tous les épitopes caractérisés sont concentrés sur un peptide de 20 acides aminés localisé dans la moitié C-terminale de la protéine NS1. Ce peptide correspond à une séquence assez bien conservée. Certains acides aminés de ce peptide sont néanmoins sérotype-dépendant, notamment ceux situés en aval de la glycine13 (cf. numérotation de la figure 1) et peuvent expliquer la restriction de 13E1B3 et 12H9G9 à la reconnaissance des sérotypes 1 et 3. De façon intéressante, cette région de NS1 à déjà été identifiée comme immunodominante pour le virus de la dengue de sérotype 2^{[11]}.
Pour les expériences de détection en SELDI-TOF MS, des anticorps monoclonaux qui pouvaient reconnaître au moins la forme monomérique de la protéine recombinante NS1 avec en plus un bon niveau de reconnaissance en ELISA ont été sélectionnés. Ces anticorps devaient reconnaître également les formes matures de la protéine NS1 qui étaient sécrétées dans un système d'infection *in vitro* de cellules Vero par le virus de la dengue. Les anticorps 13E1B3 et 12H9G9 correspondent à ces critères . Ils détectent les formes matures de la NS1 des virus de la dengue des sérotypes 1 et 3. les formes matures correspondent aux formes excrétées par les cellules dans le surnageant de culture, elles sont dépourvues de peptide signal, glycosylées et multimériques, c'est à dire au moins dimériques.

### Exemple 4 : comparaison ELISA de capture et SELDI-TOF sur matériel infecté in vitro

### Matériel et Méthodes :

Des cellules Vero (ATCC IDCCL81) cultivé en DMEM complémenté par 10% de sérum de veau foetal (DMEM-SVF) sont infectées au stade de la sub-confluence (90% de confluence). Apres contact de 90 minutes des cellules avec une des souches virales (DV1, DV2, DV3, DV4 ; Sanofi-Pasteur) diluée dans 1mL de DMEM sans sérum à une Multiplicité d'Infection (MOI) de 0.1, l'inoculum est remplacé par 10mL de milieu DMEM-SVF. Un aliquot du surnageant de culture est récolté à différents temps d'incubation post-infection(90 minutes, 6, 12, 24 et 36 heures, 5 et 7 jours). Les cellules mortes sont éliminées par une centrifugation de 5000g/5minutes. Le lysat cellulaire est obtenu après les mêmes temps d'incubation avec le virus. Dans ce cas, on ajoute 1mL de tampon de lyse (TBS-NP401%) sur le tapis cellulaire et on laisse agir 15 minutes à 4°C. Les débris sont éliminés par une centrifugation de 10 000g/5 minutes. L'infection est contrôlée par utilisation de l'ELISA Platelia™ (BioRad) qui détecte la présence de la protéine NS1 dans le surnageant de culture.
Les ELISA de capture sont effectués comme décrit dans l'exemple 3, en utilisant en capture les anticorps obtenus par immunisations de souris (cf. exemple 2).
La technologie SELDI-TOF MS a été mise au point pour l'analyse en spectrométrie de masse de mélange de protéines retenues spécifiquement par des surfaces chromatographiques. Ici, la surface chromatographique est couplée a un anticorps anti-NS1 sélectionné sur son aptitude à reconnaître la NS1 recombinante et la NS1 synthétisée *in vitro* après infection de cellules Vero. Les anticorps sont couplés sur une barrette pre-activée de type PS20 (groupe epoxy) par incubation de 0.6µg d'anticorps pendant 1 heure à température ambiante. Les barrettes sont ensuite saturées 5 minutes avec du PBS-TritonX100 0.5% et lavée une fois avec du PBS. Les échantillons (50µl), dilués 1 fois avec du PBS sont déposés sur les barrettes saturées et incubés 2 heures à température ambiante dans un bioprocesseur adapté. Finalement, elles sont lavées 3 fois avec du PBS-TritonX100 0.05%, puis 2 fois avec du PBS, afin d'éliminer le détergeant. Une matrice SPA-TFA0.5%, faite de façon extemporanée est alors ajoutée avant lecture sur l'appareil selon les instruction du fabriquant (Ciphergen Protein Biology System II (nom commercial), Biorad). Les spectres sont calibrés par le biais d'un étalon interne (ProteinChip All-in-one standard II (nom commercial), Biorad) et sont analysés par utilisation d'un logiciel approprié (ProteinChip Software V3 (nom commercial), Biorad). Tous les échantillons ont été analysés deux fois de manière indépendante. Les consommables associés à cette technologie sont commercialisés par BioRad.
Les échantillons analysés de façon comparative par les deux technologies sont les suivants :
Des lysats et des surnageant de culture prélevés à 90 minutes ; 6, 12, 24, 48 heures ; 5, 7 jours post-infection ;
De la protéine recombinante NS1 (50ng) diluée dans du plasma sain (c'est-à-dire issue d'un patient n'ayant pas la dengue);
Des lysats et des surnageants de culture de cellules Vero non infectées (7 jours post-infection) ;
Du milieu de culture.
Pour le SELDI-TOF, la valeur associée aux pics correspond à la masse rapporté à la charge (m/z) après calibration avec un standard interne. Les ELISAs de capture de la NS1 sont illustrés par des histogrammes effectués sur les mêmes échantillons. Les valeurs correspondent à la densité optique lue à 450nm (moyenne de 2 puits pour 2 expériences indépendantes).

### Résultats :

Les résultats obtenus sur du matériel infecté par le virus de sérotype 3 avec l'anticorps 13E1B3, sont présenté dans la figure 2. La même expérience mais conduite avec l'anticorps 12H9G9 donne des résultats comparables. Sur les lysats de cellules infectées on note, 12 heures après infection, l'apparition d'un pic aux environs de 43500 Daltons (Da), présent dans les prélèvements ultérieurs. Ce pic est absent des lysat de cellules non infectées. Sur les surnageant de culture, un pic d'environ 45 500 Da apparaît sans ambiguïté 24 heures après l'infection. Ce pic est absent des surnageants de culture des cellules non infectées. Un léger signal semble toutefois présent 90 minutes et 6 heures après infection : il est probablement dû aux petites quantités de protéine NS1 apportées par l'inocculum viral lors de l'infection, qui disparaissent ensuite par dégradation (12 heures après infection). La différence de masse moléculaire constatée entre protéines NS1 du lysat et du surnageant est probablement due aux modifications post-traductionelles associées à l'excrétion de la protéine (la glycosylation dans le Golgi tardif, par exemple). La protéine recombinante NS1 est détectée à une masse moléculaire voisine. Des résultats similaires ont été obtenus avec des cellules Vero infectées par le virus de la dengue de sérotype 1. Aucun signal n'est détecté sur du matériel infecté par les sérotypes 2 ou 4, ce qui est cohérent avec la nature de l'immunogène utilisé qui était proche des sérotypes 1 et 3.
Un ELISA de capture utilisé sur les mêmes échantillons confirme ces résultats : la protéine NS1 est présente dans les lysats et les surnageants après infection. Elle est détectée dans les lysats des cellules infectées avant d'être détectée dans les surnageants de culture (6 heures contre 12 heures). Comme cela ressort de la figure 2, les spécificités obtenues en utilisant un ELISA ou un SELDI TOF MS sont comparables. Cependant, dans les conditions de l'essai, en utilisant les anticorps monoclonaux 13E1B3 et 12H9G9 comme anticorps de capture, les sérotypes 2 et 4 ne sont pas détectés en SELDI TOF MS, ce qui indique que le SELDI TOF MS est une méthode spécifique des sérotypes en fonction de l'anticorps utilisé en capture.

### Exemple 5 : comparaison ELISA et SELDI-TOF sur des prélèvements humains sériés.

### Matériel et Méthodes :

Les techniques ELISA de capture et SELDI-TOF MS mises au point et décrites dans les exemples 3 et 5 sont utilisés pour évaluer de façon comparative la présence de NS1 dans les plasmas d'un patient infecté par le virus de la dengue de sérotype 3 (DV3) et prélevé 2, 3, 4, 5 jours après le début des symptômes. Ces échantillons proviennent d'une étude rétrospective conduite initialement par l'institut Louis Malardé de Papeete (Tahiti). Cette étude a été revue et approuvée par le comité éthique local. Les échantillons étaient les suivants :
Des surnageants et des lysats de culture de cellules Vero infectées par le virus de la dengue de sérotype 3 (2 jours post-infection) ;
Des surnageant et des lysats de culture de cellules Vero non infectées ;
De la protéine recombinante NS1 (50ng) diluée dans du plasma sain (c'est-à-dire issue d'un patient n'ayant pas la dengue);
Du plasma sain (c'est-à-dire issue d'un patient n'ayant pas la dengue);
Des prélèvements sériés de plasmas (infection par le virus de la dengue de sérotype 3) à 2, 3, 4, 5 jours après l'apparition des symptômes.
La valeur associée aux pics correspond à la masse rapporté à la charge (m/z) après calibration avec un standard interne. Les histogrammes illustrent des ELISAs de capture de la NS1 effectués sur les mêmes échantillons. Les valeurs correspondent à la densité optique lue à 450nm (moyenne de 2 puits pour 2 expériences indépendantes).

### Résultats :

Les résultats obtenus avec l'anticorps 13E1B3, sont présentés dans la figure 3. La même expérience, mais conduite avec l'anticorps 12H9G9 donne des résultats comparables.
Un signal est présent sur les plasmas « sérotype 3 » dès deux jours après le début des symptômes. Ce signal a une masse moléculaire d'environ 45 000 Da, comparable à celle obtenue avec une NS1 recombinante en solution (1ng/uL) ou sur des échantillons issus d'une infection de cellules Vero *in vitro.* Il n'y a aucun signal sur les plasmas sains ainsi que sur le matériel non infecté par le virus de la dengue. De la même façon un signal a pu être détecté sur deux plasmas de sérotype 1 (4 et 5 jours après le début des symptômes) mais pas sur deux plasmas de sérotype 2 (4 jours après le début des symptômes). Les résultats obtenus en SELDI-TOF MS ont été confirmés par ELISA de capture.
Le SELDI-TOF MS permet donc la détection de la NS1 dans un milieu biologique complexe à l'aide d'un seul anticorps monoclonal spécifique avec une bonne spécificité. La sensibilité est voisine, bien que légèrement inférieure, à celle d'un ELISA classique. La technique permet également de détecter des petites modifications de la protéines (par exemple, les glycosylations) et de détecter, en fonction de l'anticorps choisi, les différent sérotypes du virus à partir de la NS1.

### REFERENCES BIBLIOGRAPHIQUES

1. SB Halstead . The lancet 2007; 370: 1644-52
2. AS Leong et al. Semin.Diagn.Pathol. 2007; 24(4):227-236
3. K. Clyde et al. J. Virol. 2006; 23: 11418-11431
4. G.W Smith, J.Gen.Virol, 66, 559-571
5. P. Avirutnan et al., JID 2006; 193: 1078-108 G.W Smith[4] (J.Gen.Virol, 66, 559-571)
6. Flamand et al. J.Virol, 73(7) : 6104-6110
7. Hutchens et al. (Adv Exp Med Biol. 1998;443:23-3
8. Ashock MS et al. Vaccine, 2002
9. G. Köhler et C. Milstein, 1976, Eur J Immunol, 6, 511-519
10. R. Frank et R. Döhring, 1988, Tetrahedron, 44, 6031-6040
11. Vaughan et al. , Viral Immunol., 23(3), 259-284

## Revendications

1. Procédé pour mettre en évidence *in vitro* une infection par un virus de la dengue chez un individu qui comprend les étapes de :
mettre en contact un échantillon sanguin de l'individu avec un ligand spécifique de la protéine NS1 dudit virus de la dengue pour capturer la protéine NS1, si elle est présente dans l'échantillon sanguin, ledit ligand étant immobilisé sur un support solide,
mettre en évidence la présence de la protéine NS1 par une lecture par spectrométrie de masse, et
si la protéine NS1 est mise en évidence conclure que l'individu a été infecté par le virus de la dengue.

2. Procédé selon la revendication 1, dans lequel le ligand spécifique de la protéine NS1 est choisi parmi les anticorps, les fragments d'anticorps et les protéines d'affinité aux propriétés compétitives.

3. Procédé selon la revendication 2, dans lequel le ligand est un anticorps ou un fragment d'anticorps.

4. Procédé selon la revendication 3, dans lequel le ligand est un anticorps monoclonal ou un anticorps polyclonal hautement purifié par affinité vis-à-vis de la protéine NS1.

5. Procédé selon la revendication 1, dans lequel le ligand est spécifique de la protéine NS1 du virus de la dengue d'au moins un sérotype.

6. Procédé selon la revendication 1, dans lequel le ligand est spécifique de la protéine NS1 du virus de la dengue de deux sérotypes.

7. Procédé selon la revendication 4, dans lequel le ligand est un anticorps monoclonal spécifique de la forme monomérique, de la forme dimérique et de la forme hexamérique de la protéine NS1.

8. Procédé selon la revendication 1, dans lequel le support solide est choisi parmi une barrette, une plaque, une bille, une puce et une phase chromatographique.

## Patentansprüche

1. Verfahren zum *in vitro*-Nachweis einer Infektion durch ein Dengue-Virus bei einem Individuum, das die folgenden Schritte umfasst:
Inkontaktbringen einer Blutprobe des Individuums mit einem spezifischen Liganden des NS1-Proteins des Dengue-Virus, um das NS1-Protein, falls es in der Blutprobe vorhanden ist, einzufangen, wobei der Ligand auf einem festen Träger immobilisiert ist,
Nachweisen des Vorhandenseins des NS1-Proteins durch eine Massenspektrometriemessung und,
wenn das NS1-Protein nachgewiesen wird, Schlussfolgern, dass das Individuum durch das Dengue-Virus infiziert worden ist.

2. Verfahren nach Anspruch 1, wobei der spezifische Ligand des NS1-Proteins aus Antikörpern, Antikörperfragmenten und Affinitätsproteinen mit kompetitiven Eigenschaften ausgewählt ist.

3. Verfahren nach Anspruch 2, wobei der Ligand ein Antikörper oder ein Antikörperfragment ist.

4. Verfahren nach Anspruch 3, wobei der Ligand ein monoklonaler Antikörper oder ein durch Affinität zu dem NS1-Protein hoch gereinigter polyklonaler Antikörper ist.

5. Verfahren nach Anspruch 1, wobei der Ligand für das NS1-Protein des Dengue-Virus mindestens eines Serotyps spezifisch ist.

6. Verfahren nach Anspruch 1, wobei der Ligand für das NS1-Protein des Dengue-Virus mindestens zweier Serotypen spezifisch ist.

7. Verfahren nach Anspruch 4, wobei der Ligand ein monoklonaler Antikörper ist, der für die monomere Form, die dimere Form und die hexamere Form des NS1-Proteins spezifisch ist.

8. Verfahren nach Anspruch 1, wobei der feste Träger aus einem Muldenstreifen, einer Platte, einer Kugel, einem Chip und einer Chromatographiephase ausgewählt ist.

## Claims

1. A process for *in vitro* detection of an infection by a dengue virus in an individual, comprising the following steps:
contacting a blood sample from the individual with a ligand specific to the NS1 protein of said dengue virus, to capture NS1 protein if it is present in the blood sample, said ligand being immobilised on a solid support,
detecting the presence of NS1 protein via a mass spectrometry reading, and
if NS1 protein is detected, concluding that the individual has been infected by dengue virus.

2. The process according to claim 1, wherein the ligand specific to the NS1 protein is chosen from the antibodies, antibody fragments and affinity proteins with competitive properties.

3. The process according to claim 2, wherein the ligand is an antibody or an antibody fragment.

4. The process according to claim 3, wherein the ligand is a monoclonal antibody or a polyclonal antibody highly purified by affinity to NS1 protein.

5. The process according to claim 1, wherein the ligand is specific to the NS1 protein of dengue virus of at least one dengue virus serotype.

6. The process according to claim 1, wherein the ligand is specific to the NS1 protein of dengue virus of two dengue virus serotypes.

7. The process according to claim 4, wherein the ligand is a monoclonal antibody specific to the monomeric form, the dimeric form and the hexameric form of the NS1 protein.

8. The process according to claim 1, wherein the solid support is chosen from a well strip, a plate, a ball, a chip and a chromatographic phase.
